# EUROPÄISCHE PATENTANMELDUNG

(11) **EP 3 835 758 A1**
(43) Veröffentlichungstag der Anmeldung: **16.06.2021**
(21) Anmeldenummer: 20213088.6
(22) Anmeldetag: 10.12.2020
(51) Int. Cl.: G01N 21/31, G01N 21/94, A61L 2/10, C02F 1/32, G01N 33/18, G01N 21/33, G01N 21/3577, G01N 21/53, G01N 21/90

(54) **VERFAHREN UND VORRICHTUNG ZUR DESINFIZIERUNG UND MESSUNG EINES FLUIDS**

(30) Priorität: 13.12.2019 AT 510902019
(71) Anmelder: Hörmann, Richard, 6020 Innsbruck (AT)
(72) Erfinder: Hörmann, Richard, 6020 Innsbruck (AT)
(74) Vertreter: Torggler & Hofinger Patentanwälte

(57) **Zusammenfassung**

Verfahren zur Desinfizierung und Messung eines Fluids mittels Licht, wobei folgende Schritte durchgeführt werden:
- Emittieren eines Lichts aus einer LED-Lichtquelleneinrichtung (1) mit einer ersten Intensität,
- Messen der Intensität des mit der ersten Intensität emittierten Lichts nach einer Transmission des Lichts durch das Fluid (2),
- Emittieren eines Lichts aus einer LED-Lichtquelleneinrichtung (1) mit einer zweiten Intensität,
- Messen der Intensität des mit der zweiten Intensität emittierten Lichts nach einer Transmission des Lichts durch das Fluid (2),
- Bestimmen von Absorptionseigenschaften und/oder Trübe und/oder spektraler Zusammensetzung des Lichts nach einer Transmission des Lichts durch das Fluid (2) und/oder einer Verunreinigung einer Schutzeinrichtung aus den gemessenen Intensitäten, und
- Desinfizierung des Fluids (2) durch die Transmission des emittierten Lichts durch das Fluid (2).

Vorrichtung zur Durchführung des Verfahrens.

## Beschreibung

Die Erfindung betrifft ein Verfahren zur Desinfizierung und Messung eines Fluids mittels LED-Licht. Weiters betrifft die Erfindung eine Vorrichtung zur Desinfizierung und Messung eines Fluids mittels eines erfindungsgemäßen Verfahrens.

Gemäß dem Stand der Technik ist es bekannt, mikrobiologisch kontaminierte Stoffe, wie z.B. Trinkwasser oder auch gereinigtes Abwasser, durch Bestrahlung mit ultraviolettem Licht (UV-Licht) mit einer Wellenlänge von 254nm zu entkeimen. Im Stand der Technik wird zur Erzeugung eines UV-Lichts meist auf Gasentladungslampen zurückgegriffen, die das UV-Licht über Stoßionisation in einer Quecksilberdampffüllung erzeugen.

Da zur spektrale Analyse von Stoffen die Absorptionseigenschaften der zu messenden Stoffe verwendet werden, können mittels UV-Licht nicht nur eine Desinfektion eines Stoffs, insbesondere eines Fluids, erfolgen sondern auch quantitative Aussagen über die Qualität und Zusammensetzung des zu messenden Fluids getroffen werden.

Die anmeldereigene AT 504 205 A4 beschreibt ein Verfahren zur Desinfektion und Messung eines Fluids mittels UV-Licht. Zur Messung des Fluids ist in der AT 504 205 A4 eine Messkammer vorgesehen, welche in einem ersten Betriebsmodus mit einem zu messenden Fluid und in einem zweiten Betriebsmodus mit einem Referenzmedium befüllbar ist. In einem ersten Betriebsmodus wird das in der Messkammer befindliche Fluid und in dem zweiten Betriebsmodus das Referenzmedium gemessen. Anschließen können aus den Messwerten des Fluids und den Messwerten des Referenzmediums Absorptionseigenschaften usw. des Fluids bestimmt werden

Nachteilig an einem solchen Verfahren ist die Notwendigkeit einer eigenen Messkammer und eines Referenzmediums. Dies wirkt sich negativ aus auf:
- den benötigten Bauraum: eine Nachrüstung bestehender UV-Anlagen ist nicht möglich
- die Geschwindigkeit mit welcher Messungen durchgeführt werden können, und
- die Komplexität der Messvorrichtung: diese steigt und erhöht die Fehleranfälligkeit dieser Vorrichtung.

Es ist nun weiters aus dem Stand der Technik bekannt, UV-LED-Lichtquellen zur Desinfizierung und Messung von Fluiden einzusetzen. Hierbei kommen sogenannte UVC-LEDs zum Einsatz, welche ein Emissionsspektrum von 100nm bis 280nm aufweisen.

Auch das in der AT 504 205 A4 genannte Verfahren kann grundsätzlich mit UV-LED-Lichtquellen durchgeführt werden. Dies würde allerdings die oben angeführten Probleme nicht beheben und somit nur einen höheren Kostenaufwand nach sich ziehen.

Als LED werden in dieser Anmeldung sowohl Leuchtdioden als auch Laserdioden verstanden.

Die Aufgabe der vorliegenden Erfindung besteht darin, die Nachteile des Stands der Technik zu beheben und ein gegenüber dem Stand der Technik verbessertes Verfahren zur Desinfizierung und Messung eines Fluids anzugeben. Eine weitere Aufgabe besteht darin eine Vorrichtung für ein erfindungsgemäßes Verfahren zur Desinfizierung und Messung eines Fluids anzugeben.

Diese Aufgaben werden gelöst durch die Merkmale der unabhängigen Ansprüche 1 und 10.

In Bezug auf ein erfindungsgemäßes Verfahren ist es also vorgesehen, dass das Verfahren folgende Schritte umfasst:
- Emittieren eines Lichts aus einer LED-Lichtquelleneinrichtung mit einer ersten Intensität,
- Messen der Intensität des mit der ersten Intensität emittierten Lichts nach einer Transmission des Lichts durch das Fluid,
- Emittieren eines Lichts aus einer LED-Lichtquelleneinrichtung mit einer zweiten Intensität,
- Messen der Intensität des mit der zweiten Intensität emittierten Lichts nach einer Transmission des Lichts durch das Fluid,
- Bestimmen von Absorptionseigenschaften und/oder Trübe und/oder spektraler Zusammensetzung des Lichts nach einer Transmission durch das Fluid und/oder einer Verunreinigung einer Schutzeinrichtung zur Aufnahme eines Fluids aus den gemessenen Intensitäten, und
- Desinfizierung des Fluids durch die Transmission des emittierten Lichts durch das Fluid.

Hinsichtlich einer erfindungsgemäßen Vorrichtung ist es vorgesehen, dass die Vorrichtung folgendes umfasst:
- wenigstens einen Behälter zur Aufnahme eines Fluids,
- wenigstens eine LED-Lichtquelleneinrichtung,
- wenigstens eine Messeinrichtung zur Messung eines von der LED-Lichtquelleneinrichtung abgestrahlten Lichts nach einer Transmission des Lichts durch das Fluid, und
- wenigstens eine Kontrolleinheit zur Steuerung und/oder Regelung der LED-Lichtquelleneinrichtung und/oder der Messeinrichtung.

Bei einem erfindungsgemäßen Verfahren und einer erfindungsgemäßen Vorrichtung müssen also zur Bestimmung von Absorptionseigenschaften oder Trübe eines Fluids keine Messkammern oder Teilstrecken im Fluid konstruiert werden und es muss auch kein Referenzmedium vorhanden sein. Für die Messung reicht es aus, wenn das Licht dieselbe Wegstrecke durch das Fluid nimmt wie für die Desinfektion.

Dadurch wird einerseits der benötigte Bauraum im Vergleich zu herkömmlichen UV-Anlagen reduziert und die Geschwindigkeit, mit welcher Messungen durchgeführt werden können, erhöht. Andererseits werden die Komplexität und somit auch die Fehleranfälligkeit einer erfindungsgemäßen Vorrichtung verringert.

Weiters kann durch ein erfindungsgemäßes Verfahren die LED Technologie optimal ausgenützt werden.

Weitere vorteilhafte Ausführungen der Erfindung sind in den abhängigen Ansprüchen definiert.

Bei einem erfindungsgemäßen Verfahren kann vorgesehen sein, dass das Licht mit der ersten Intensität und das Licht mit der zweiten Intensität von derselben LED-Lichtquelleneinrichtung emittiert werden. Eine LED-Lichtquelleneinrichtung kann dabei grundsätzlich jede beliebige Zahl an LEDs aufweisen.

Weiters kann vorgesehen sein, dass eine Änderung der Intensität eines mit der ersten Intensität emittierten Lichts auf eine Intensität eines mit der zweiten Intensität emittierten Lichts durch eine Pulsweitenmodulation der wenigstens einen LED-Lichtquelleneinrichtung erfolgt.

Es wird also eine elektrische Größe, beispielsweise die Spannung, der Strom oder die Leistung, welche an der LED-Lichtquelleneinrichtung anliegt, moduliert. Dabei ist der Wert der zu modulierenden Größe zu einem ersten Zeitpunkt größer als zu einem zweiten Zeitpunkt. Da die von der LED-Lichtquelleneinrichtung an das Fluid abgegebene Strahlungsintensität proportional zu der modulierten elektrischen Größe ist, ist der von der Messeinrichtung gemessene Wert der Intensität zu einem ersten Zeitpunkt größer als zu einem zweiten Zeitpunkt.

Bei der Transmission des Lichts durch das Fluid desinfiziert das UV-Licht das Fluid und trifft nach der Transmission auf die Messeinrichtung, wobei zu dem ersten Zeitpunkt eine erste - größere - Intensität und zu einem zweiten Zeitpunkt eine zweite - kleinere - Intensität auf die Messeinrichtung trifft. Die Messeinrichtung gibt anschließend zu den gemessenen Intensitäten des Lichtes proportionale Werte einer elektrischen Größe - Messwerte - aus.

Je nach Beschaffenheit des Fluids kommt es bei der Transmission des Lichts durch das Fluid zu einer Schwächung der Intensität. Die gesamte Schwächung setzt sich zusammen aus einem Anteil, der auf die gelösten Substanzen und einem, der auf die ungelösten Substanzen im Fluid zurückgeht. Die quantitative Bestimmung beider Anteile, die für die Beurteilung der Qualität des Fluids entscheidend ist, geschieht durch Auswertung der Messwerte unter Zuhilfenahme des Gesetzes von Lambert und Beer.

Emittiert die LED-Lichtquelleneinrichtung nur Licht im UV-Bereich, so ergeben sich aus dieser Auswertung unter anderem:
- Die Gesamtbelastung des Fluids in Form des Spektralen Schwächungskoeffizienten (SSK).
- Die UV-Transmission.

Es kann weiters eine einmalige Kalibrierung einer erfindungsgemäßen Vorrichtung vorgesehen sein, bei welcher eine Position der Messeinrichtung verändert wird.

Da eine Intensität eines mit einer zweiten Intensität zu einem zweiten Zeitpunkt emittierten Lichts kleiner als eine erste Intensität ist, ist zu diesem zweiten Zeitpunkt die Strahlungsleistung geringer als zu einem ersten Zeitpunkt. Die für eine Desinfektion wichtige, gleichbleibende Strahlungsleistung kann so erreicht werden, dass zu einem ersten Zeitpunkt eine Überkompensation der Leistung stattfindet, die die bei einem zweiten Zeitpunkt vorhandene Unterkompensation ausgleicht.

Es kann zusätzlich vorgesehen sein, eine LED-Lichtquelleneinrichtung mit Emissionsspektren im VIS-Bereich einzusetzen, die eine Trübekompensation ermöglichen.

Aus einer Anwendung des gegenständlichen Verfahrens auf den VIS-Bereich ergeben sich unter anderem folgende Parameter:
- Der Anteil an gelösten organischen Stoffe in Form der Summenparameter:
   ∘ Spektraler Absorptionskoeffizient (SAK),
   ∘ Gesamter organischer Kohlenstoff/total organic carbon (TOC), und
   ∘ Chemischer Sauerstoffbedarf (CSB)
- Der Anteil an ungelösten Stoffen in Form der Trübe.

Es kann auch vorgesehen sein, dass eine LED-Lichtquelleneinrichtung sowohl UVC-LEDs als auch UVB-LEDs mit einem Emissionsspektrum von 210nm bis 315nm und/oder UVA-LEDs mit einem Emissionsspektrum von 315nm bis 380nm umfasst.

Zusammen mit einer oder mehreren Messeinrichtungen, welche auf die entsprechenden Spektren der UVC- bzw. UVB- bzw. UVA-LEDs kalibriert sind, ergibt sich ein einfaches Spektrometer, das die spektrale Zusammensetzung des Lichts nach einem Durchgang durch das Fluid genauer analysieren und die im Fluid vorhandenen Stoffe anhand ihrer spektralen "Fingerprints" ermitteln kann.

Die "Fingerprints" ergeben sich durch Anwendung des erfindungsgemäßen Verfahrens auf jedes der von den LEDs abgegebenen Emissionsspektren. Demnach lässt sich für eine bestimmte Wellenlänge der Wert des SSK bei dieser Wellenlänge und mittels der Trübekompensation der Wert des SAK bei dieser Wellenlänge ermitteln.

Die "Fingerprints" ermöglichen eine genauere Berechnung von Summenparameter wie dem TOC oder dem CSB. Zwischen dem SAK254 (SAK bei einer Wellenlänge von 254nm) und diesen Summenparametern gibt es kein konstantes Verhältnis. Das Verhältnis ist von der Zusammensetzung des zu messenden Fluids abhängig. Mittels einer spektralen Analyse und statistischer Vergleiche mit Norm-Werten kann aus dem SAK254 der entsprechende CSB oder TOC Wert eines Fluids spezifischer Zusammensetzung ermittelt werden.

Wird eine solche LED-Lichtquelleneinrichtung mit UVC-, UVB- und UVA-LEDs verwendet, so ist zu berücksichtigen, dass LEDs mit Emissionsspektren ungleich 254nm vorzugsweise nur an den Stellen angeordnet werden sollten, an welchen gegenüberliegend eine Messeinrichtung platziert ist, da Licht mit einer Wellenlänge ungleich 254nm keine Desinfektion bewirkt.

An den Stellen, an denen UVB- und UVA-LEDs vorhanden sind, kommt es zu einem kleinen Verlust an Desinfektionsleistung, weil die Dichte an UVC-LEDs dort geringer ist. Ist eine Kompensation dieses Verlustes erforderlich, kann das durch eine Überkompensation der den UVC-LEDs insgesamt zugeführten elektrischen Energie erfolgen.

Es kann weiters vorgesehen sein, dass zur Bestimmung der spektralen Zusammensetzung des Lichts nach einer Transmission durch das Fluid optische Eigenschaften, vorzugsweise Frequenz oder Wellenlänge, eines von der wenigstens einen LED-Lichtquelleneinrichtung emittierten Lichts moduliert wird, wobei durch das in seinen optischen Eigenschaften modulierte emittierte Licht in Verbindung mit wenigstens einer Messeinrichtung zur Messung der Intensitäten ein Spektrometer gebildet wird.

Die Modulation der Frequenz und demnach der Wellenlänge und einer Amplitude einer LED, insbesondere einer Laserdiode, kann dabei über eine piezoelektrisch herbeigeführte Längenänderung eines Resonators der zu modellierenden LED, insbesondere Laserdiode, erfolgen.

Für Leuchtdioden ist weiters auch zumindest der experimentelle Nachweis gelungen, dass im sichtbaren Bereich (VIS-Bereich) eine Veränderung der Wellenlänge des emittierten Lichtes über einen Diodenstrom möglich ist. Wird der Diodenstrom über PWM geregelt, ist die Farbe als Funktion der Stromstärke, der PWM-Frequenz und des Duty-Cycles modellier- und einstellbar.

Unter Berücksichtigung dieser Modellierung ist dann die Modulation des LED-Lichtes nicht mehr nur durch die Modulation einer elektrischen Eingangsgröße zu erreichen, sondern durch eine komplexe Modulation mehrerer Größen.

Mithilfe solcher in den optischen Eigenschaften modulierbaren LEDs lässt sich zusammen mit der beschriebenen Konstellation aus LED-Lichtquelleneinrichtung, einem Fluid und wenigstens einer Messeinrichtung ein Spektrometer realisieren, das über den gesamten UV-Bereich - gegebenenfalls auch im VIS- oder IR-Bereich - die Absorptionspeaks des zu messenden Fluids misst und daraus ein detailliertes spektrales Profil des Fluids erstellt.

Der so einstellbare Wellenlängenbereich reicht dabei von 190nm bis 800nm.

Durch Anwendung des erfindungsgemäßen Verfahrens können bei jeder beliebigen Frequenz der zugehörige SSK und der zugehörige SAK bestimmt werden.

Im Gegensatz zu dem mittels UVC-, UVB- und UVA-LEDs realisiertem einfachen Spektrometer, bei dem das Fluid einigen wenigen, diskreten Wellenlängen ausgesetzt wird, handelt es sich hier um ein vollwertiges Spektrometer, das den gesamten UV, VIS- und/oder IR-Bereich abdeckt und somit exakte "Fingerprints" der im Fluid enthaltenen Stoffe ermittelt. Diese Ausführungsform stellt also eine weitere deutliche Optimierung in der Bestimmung der Summenparameter wie beispielsweise des CSB oder des TOC dar.

Es kann auch vorgesehen sein, mittels räumlicher Versetzung von UV-LEDs oder gegebenenfalls VIS- oder IR-LEDs und individueller Ansteuerung der räumlich versetzten LEDs UV-Licht in einer zeitlich und örtlich variierenden Intensität an die Messeinrichtung abzustrahlen. Durch eine statistische Auswertung der durch die zeitlich und räumlich variierende Intensität entstehenden Signalform der durch die Messeinrichtung gemessenen Messwerte, kann eine Verunreinigung einer Schutzeinrichtung detektiert werden.

Weitere Einzelheiten und Vorteile der Erfindung werden anhand der Figurenbeschreibung unter Bezugnahme auf die Zeichnungen im Folgenden näher erläutert. Darin zeigen:
- Fig. 1: eine Ausführungsform einer erfindungsgemäßen Vorrichtung in einer schematischen Darstellung,
- Fig. 2a: eine weitere Ausführungsform einer erfindungsgemäßen Vorrichtung in einer schematischen Draufsicht,
- Fig. 2b: die Ausführungsform einer erfindungsgemäßen Vorrichtung nach Figur 2a in einer schematischen Teilschnittdarstellung,
- Fig. 3a: eine weitere Ausführungsform einer erfindungsgemäßen Vorrichtung in einer schematischen Schnittansicht,
- Fig. 3b: die Ausführungsform einer erfindungsgemäßen Vorrichtung nach Figur 3a in einer schematischen Seitenansicht, und
- Fig. 4: eine weitere Ausführungsform einer erfindungsgemäßen Vorrichtung in einer schematischen Draufsicht.

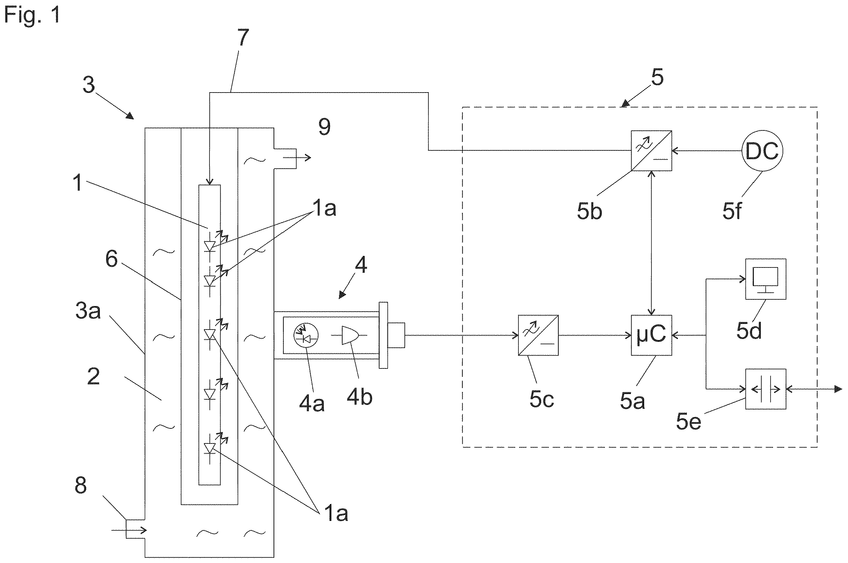

In Figur 1 ist eine Ausführungsform einer erfindungsgemäßen Vorrichtung in einer schematischen Darstellung abgebildet. Es ist ein Behälter 3 zur Aufnahme eines Fluids 2 mit einem Rand 3a und einem Zufluss 8 und einem Abfluss 9 erkennbar.

Am Rand 3a des Behälters 3 ist eine Messeinrichtung 4 angeordnet, welche einen Lichtsensor 4a und einen Verstärker 4b umfasst, welcher ein vom Lichtsensor 4a ausgegebenes Messsignal verstärkt.

Innerhalb des Behälters 3 ist eine LED-Lichtquelleneinrichtung 1 mit einer Vielzahl an LEDs 1a angeordnet. Die LED-Lichtquelleneinrichtung 1 ist von einer Schutzeinrichtung 6 in Form eines Quarzrohres vor einem Eindringen von Fluid 2 geschützt und über eine elektrische Leitung 7 mit einer Kontrolleinheit 5 verbunden.

Die elektrische Leitung 7 ist dabei an einen Modulator 5b angeschlossen, welcher ein PWM-Signal an die LED-Lichtquelleneinrichtung 1 senden kann, und wiederum mit einer Energiequelle 5f verbunden ist.

Die Kontrolleinheit 5 umfasst weiters eine Anzeigeeinheit 5d, eine Schnittstelle 5e zur Kommunikation mit peripheren Geräten, einen Microcontroller 5a sowie einen Demodulator 5c.

Der Demodulator 5c ist mit der Messeinrichtung 4 verbunden und konvertiert ein von der Messeinrichtung 4 empfangenes PWM-Signal in ein DC-Signal, welches anschließend an den Microcontroller 5a weitergegeben wird. Der Microcontroller 5a errechnet aus den von der Messeinrichtung 4 empfangenen Messsignalen die gewünschten Parameter.

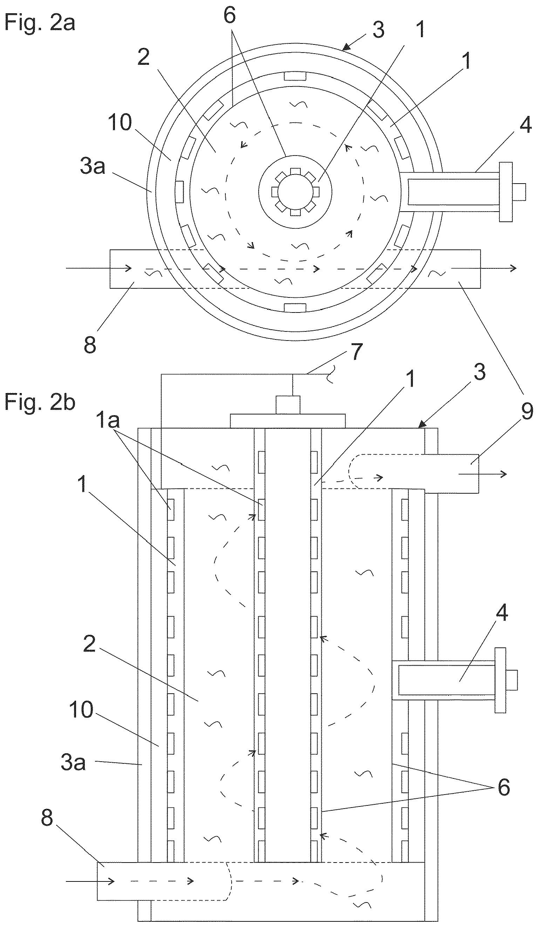

Die Figur 2a zeigt eine weitere Ausführungsform einer erfindungsgemäßen Vorrichtung in einer schematischen Draufsicht, Figur 2b zeigt diese Ausführungsform in einer Teilschnittdarstellung. Es ist erkennbar, dass sowohl im Wesentlichen mittig in dem Behälter 3 als auch am Rand 3a des Behälters 3 jeweils eine LED-Lichtquelleneinrichtung 1 angeordnet ist.

In Kombination mit einem tangentialen Zulauf 8 und Ablauf 9 des Fluids 2, der eine mittlere Aufenthaltswahrscheinlichkeit der Fluidteilchen des Fluids 2 in der Mitte zwischen den als Quarzrohren ausgeführten Schutzeinrichtungen 6 bewirkt, ergibt sich eine Verdopplung der Desinfektionsleistung.

Es sind weiters die am Rand 3a angeordnete Messeinrichtung 4, die elektrische Leitung 7 zum Ansteuern der LED-Lichtquelleneinrichtung 1 sowie ein LED-Träger 10 erkennbar.

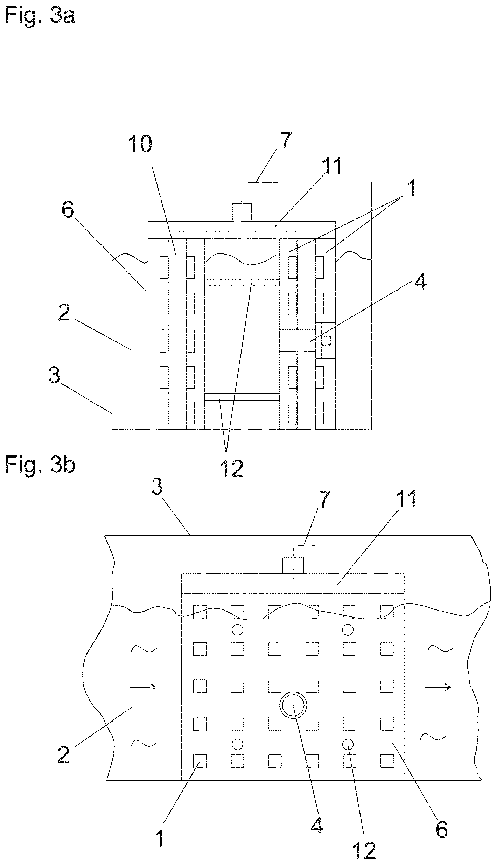

Die Figuren 3a und 3b zeigen eine weitere Ausführungsform der gegenständlichen Erfindung. Bei dieser Ausführungsform ist eine erfindungsgemäße Vorrichtung in einem Schacht 13 oder Kanal angeordnet.

Die UV-LED-Lichtquellen 1 sind dabei parallel zueinander auf LED-Trägern 10 angeordnet und durch als Quarzplatten ausgebildete Schutzeinrichtungen 6 vor dem Fluid 2 geschützt.

Die LED-Träger 10 sind über Verbindungsstäbe 12 miteinander verbunden. Es ist weiters eine Deckplatte 11 erkennbar in welcher auch die elektrische Leitung 7 zu den LED-Lichtquelleneinrichtungen 1 geführt ist.

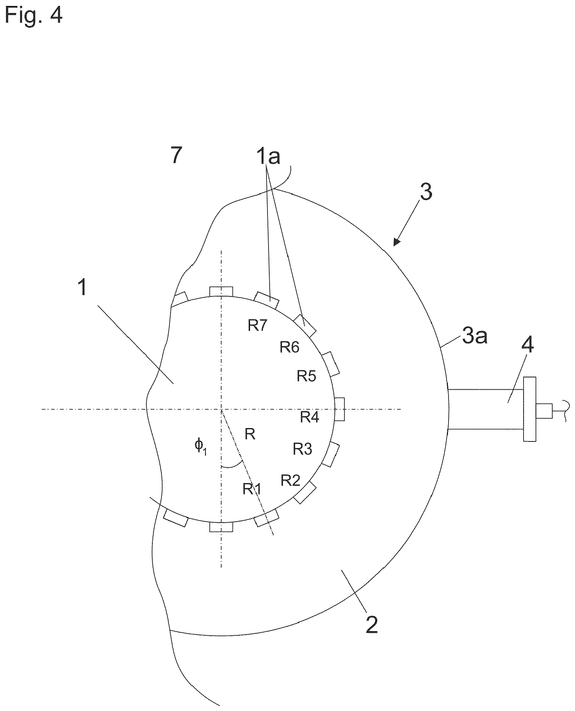

Die Figur 4 zeigt eine schematische Draufsicht eines weiteren Ausführungsbeispiels einer erfindungsgemäßen Vorrichtung. Dabei ist eine Mehrzahl 1b an LEDs 1a jeweils in einer Reihe R1 bis R7 angeordnet. Diese Reihen R1 bis R7 sind dabei
gegenüber der Messeinrichtung 4 so angeordnet, dass jede von ihnen in einem unterschiedlichen Winkel zur Messeinrichtung 4 steht.

Jede Reihe R1 bis R7 kann individuell angesteuert werden und ihr Licht in einer zeitlich und örtlich variierenden Intensität an die Messeinrichtung 4 abstrahlen. Ist die LED-Lichtquelleneinrichtung 1 röhrenförmig ausgebildet, so sind die Reihen R1 bis R7 in einem Halbkreis gegenüber der Messeinrichtung 4 positioniert. Somit entsteht eine wandernde Intensitätswelle, die von der Messeinrichtung 4 als elektrisches Signal ausgegeben wird.

Durch eine statistische Auswertung der durch die zeitlich und räumlich variierende Intensität entstehenden Signalform der durch die Messeinrichtung 4 gemessenen Messwerte, kann eine Verunreinigung einer Schutzeinrichtung 6 detektiert werden.

Neben diesem Ausführungsbeispiel sind auch andere geometrische Anordnungen der Reihen R1 bis R7 und auch eine andere Anzahl an Reihen denkbar.

### Bezugszeichenliste:

- 1: LED-Lichtquelleneinrichtung
1a LEDs
1b Mehrzahl an EDs
- 2: Fluid
- 3: Behälter f. Fluid
3a Rand
- 4: Messeinrichtung
4a Lichtsensor
4b Verstärker
- 5: Kontrolleinheit
5a Microcontroller
5b Modulator
5c Demodulator
5d Anzeigeeinheit
5e Schnittstelle
5f Energiequelle
- 6: Schutzeinrichtung
- 7: El. Leitung
- 8: Zulauf
- 9: Ablauf
- 10: LED-Träger
- 11: Deckplatte
- 12: Verbindungsstäbe
- 13: Schacht

## Patentansprüche

1. Verfahren zur Desinfizierung und Messung eines Fluids mittels Licht einer LED-Lichtquelleneinrichtung, **gekennzeichnet durch** folgende Schritte:
- Emittieren eines Lichts aus einer LED-Lichtquelleneinrichtung (1) mit einer ersten Intensität,
- Messen der Intensität des mit der ersten Intensität emittierten Lichts nach einer Transmission des Lichts durch das Fluid (2),
- Emittieren eines Lichts aus einer LED-Lichtquelleeinrichtung (1) mit einer zweiten Intensität,
- Messen der Intensität des mit der zweiten Intensität emittierten Lichts nach einer Transmission des Lichts durch das Fluid (2),
- Bestimmen von Absorptionseigenschaften und/oder Trübe und/oder spektraler Zusammensetzung des Lichts nach einer Transmission durch das Fluids (2) und/oder einer Verunreinigung einer Schutzeinrichtung (6) aus den gemessenen Intensitäten, und
- Desinfizierung des Fluids (2) durch die Transmission des von der LED-Lichtquelleneinrichtung (1) emittierten Lichts durch das Fluid (2).

2. Verfahren nach Anspruch 1, wobei die LED-Lichtquelleneinrichtung (1) mindestens eine ein ultraviolettes Licht emittierende LED aufweist, wobei vorzugsweise die LED-Lichtquelleneinrichtung (1) ausschließlich aus UV-Lichtquellen gebildet wird.

3. Verfahren nach einem der Ansprüche 1 oder 2, wobei die LED-Lichtquelleneinrichtung (1) mindestens eine VIS- oder eine IR-Lichtquelle umfasst.

4. Verfahren nach einem der Ansprüche 1 bis 3, **dadurch gekennzeichnet, dass** das Licht mit der ersten Intensität und das Licht mit der zweiten Intensität von derselben wenigstens einen LED-Lichtquelleneinrichtung (1) emittiert werden.

5. Verfahren nach einem der Ansprüche 1 bis 4, **dadurch gekennzeichnet, dass** eine Änderung der Intensität eines mit der ersten Intensität emittierten Lichts auf eine Intensität eines mit der zweiten Intensität emittierten Lichts durch eine Änderung zumindest einer elektrischen Größe, vorzugsweise durch eine Pulsweitenmodulation, der wenigstens einen LED-Lichtquelleneinrichtung (1) erfolgt.

6. Verfahren nach einem der Ansprüche 1 bis 5, **dadurch gekennzeichnet, dass** die aus den gemessenen Intensitäten bestimmten Parameter zumindest einen der nachfolgenden umfassen:
- Spektraler Schwächungskoeffizient (SSK)
- UV-Transmission (UVT)
- Spektraler Absorptionskoeffizient (SAK)
- Gesamter organischer Kohlenstoff/total organic carbon (TOC)
- Chemischer Sauerstoffbedarf (CSB)
- Trübe

7. Verfahren nach einem der Ansprüche 1 bis 6, **dadurch gekennzeichnet, dass** die wenigstens eine LED-Lichtquelleneinrichtung (1) zur Bestimmung der spektralen Zusammensetzung des Lichts nach einer Transmission durch das Fluid (2) Licht in unterschiedlichen Emissionsspektren emittiert, wobei durch das in unterschiedlichen Emissionsspektren emittierte Licht in Verbindung mit wenigstens einer Messeinrichtung (4) zur Messung der Intensitäten ein Spektrometer gebildet wird.

8. Verfahren nach einem der Ansprüche 1 bis 6, **dadurch gekennzeichnet, dass** zur Bestimmung der spektralen Zusammensetzung des Lichts nach einer Transmission durch das Fluid (2) optische Eigenschaften - beispielsweise Frequenz und Amplitude - eines von der wenigstens einen LED-Lichtquelleneinrichtung (1) emittierten Lichts moduliert wird, wobei durch das in seinen optischen Eigenschaften Wellenlänge modulierte emittierte Licht in Verbindung mit wenigstens einer Messeinrichtung (4) zur Messung der Intensitäten ein Spektrometer gebildet wird.

9. Verfahren nach einem der Ansprüche 1 bis 8, **dadurch gekennzeichnet, dass** die wenigstens eine LED-Lichtquelleneinrichtung (1) zur Bestimmung einer Verunreinigung der Schutzeinrichtung (6) Licht mit einer zeitlich und räumlich veränderlichen Intensität emittiert, welches anschließend gemessen und ausgewertet wird.

10. Vorrichtung zur Desinfizierung und Messung eines Fluids (2) mit einem Verfahren nach Anspruch 1, umfassend
- wenigstens einen Behälter (3) zur Aufnahme eines Fluids (2),
- wenigstens eine - vorzugsweise eine Vielzahl an LEDs (a) aufweisenden - LED-Lichtquelleneinrichtung (1),
- wenigstens eine Messeinrichtung (4) zur Messung eines von der LED-Lichtquelleneinrichtung (1) abgestrahlten Lichts nach einer Transmission des Lichts durch das Fluid (2), und
- wenigstens eine Kontrolleinheit (5) zur Steuerung und/oder Regelung der wenigstens einen LED-Lichtquelleneinrichtung (1) und/oder der Messeinrichtung (4).

11. Vorrichtung nach Anspruch 10, **dadurch gekennzeichnet, dass** die Kontrolleinheit (5) zumindest einen Microcontroller (5a) und/oder zumindest einen Modulator (5b) zur Ansteuerung der wenigstens einen LED-Lichtquelleneinrichtung (1) und/oder zumindest einen der Messeinrichtung (4) nachgeschalteten Demodulator (5c) umfasst, wobei die Kontrolleinheit (5) vorzugsweise zumindest eine Anzeigeeinheit (5d) und/oder zumindest eine Schnittstelle (5e), vorzugsweise Ethernet, zur Kommunikation mit peripheren Geräten aufweist.

12. Vorrichtung nach einem der Ansprüche 10 bis 11, **dadurch gekennzeichnet, dass** die Messeinrichtung (4) zumindest einen Lichtsensor (4a), vorzugsweise eine SiC basierte UV-Photodiode, und zumindest einen Verstärker (4b) zur Verstärkung eines Messsignals des Lichtsensor (4a) aufweist, wobei die Messeinrichtung (4) vorzugsweise dazu ausgebildet ist, sowohl UV-Licht als auch VIS-Licht und/oder IR-Licht zu messen.

13. Vorrichtung nach einem der Ansprüche 10 bis 12, **dadurch gekennzeichnet, dass** die wenigstens eine LED-Lichtquelleneinrichtung (1) im Wesentlichen mittig in dem Behälter (3) zur Aufnahme des Fluids (2) und die Messeinrichtung (4) außerhalb des Behälters (3) zur Aufnahme eines Fluids (2) angeordnet sind.

14. Vorrichtung nach einem der Ansprüche 10 bis 13, **dadurch gekennzeichnet, dass** zumindest zwei LED-Lichtquelleneinrichtungen (1) vorgesehen sind, wobei eine erste der zumindest zwei LED-Lichtquelleneinrichtungen (1) vorzugsweise im Wesentlichen mittig in dem Behälter (3) zur Aufnahme des Fluids (2) und eine zweite der beiden LED-Lichtquelleneinrichtungen (1) vorzugsweise im Wesentlichen an einem Rand (3a) des Behälters (3) zur Aufnahme des Fluids (2) angeordnet sind.

15. Vorrichtung nach einem der Ansprüche 10 bis 14, **dadurch gekennzeichnet, dass** zumindest zwei im Wesentlichen plattenförmige LED-Lichtquelleneinrichtungen (1) vorgesehen sind, wobei die zumindest zwei LED-Lichtquelleneinrichtungen (1) vorzugsweise parallel angeordnet sind.

16. Vorrichtung nach einem der Ansprüche 10 bis 15, **dadurch gekennzeichnet, dass** die wenigstens eine LED-Lichtquelleneinrichtungen (1) UVC-LEDs und/oder VIS-LEDs aufweist, wobei die wenigstens eine LED-Lichtquelle (1) zusätzlich zu den UVC-LEDs und/oder VIS-LEDs vorzugsweise weitere LEDs mit diskreten Emissionsspektren aufweist.

17. Vorrichtung nach einem der Ansprüche 10 bis 16 **dadurch gekennzeichnet, dass** mittels Änderung von elektrischen, vorzugsweise des Diodenstroms, oder mechanischen, vorzugsweise einer Resonatorlänge, Eigenschaften einer LED optische Eigenschaften, vorzugsweise Frequenz oder Amplitude, des von der LED-Lichtquelleneinrichtung (1) emittierten Lichts eingestellt und/oder moduliert werden können.

18. Vorrichtung nach einem der Ansprüche 10 bis 17, **dadurch gekennzeichnet, dass** wenigstens eine zumindest bereichsweise lichtdurchlässige Schutzeinrichtung (6), vorzugsweise ein Quarzrohr, vorgesehen ist, welche die wenigstens eine LED-Lichtquelleneinrichtung (1) umschließt und die wenigstens eine LED-Lichtquelleneinrichtung (1) somit vor einem Eindringen des Fluids (2) in die wenigstens eine LED-Lichtquelleneinrichtung (1) schützt.
